# EUROPEAN PATENT SPECIFICATION

(11) **EP 3 806 722 B1**
(45) Date of publication and mention of the grant of the patent: **13.12.2023**
(21) Application number: 19732089.8
(22) Date of filing: 12.06.2019
(51) Int. Cl.: A61B 5/01, A61B 5/08

(54) **APPARATUS FOR SENSING**
MESSVORRICHTUNG
APPAREIL DE DÉTECTION

(30) Priority: 13.06.2018 GB 201809705
(43) Date of publication of application: 21.04.2021
(73) Proprietor: Braintrain2020 Limited, Sheffield, South Yorkshire S3 7HQ (GB)
(72) Inventor: MILLS, Richard, Sheffield South Yorkshire S6 1RU (GB); VAN DE WERKEN, Maan, Sheffield South Yorkshire S10 4NF (GB); HALL, Richard, Harrogate Yorkshire HG3 5SN (GB); FLOOD, Tim, Durham TS18 5LB (GB); IRONMONGER, Paul, Doncaster, South Yorkshire DN12 3QL (GB)
(74) Representative: HGF
(86) International application number: PCT/GB2019/051619
(87) International publication number: WO 2019/239124

(56) References cited:
- WO-A1-2011/157873
- US-A- 4 945 919
- US-A1- 2017 367 651

## Description

### TECHNICAL FIELD

The present invention relates to apparatus and methods for sensing and determining respiratory information about a sleeping user.

### BACKGROUND

Sleep disorders, including difficulties in either falling asleep or remaining asleep, are increasingly common; one in three adults in the UK are reported to suffer from a sleep disorder of some kind. A lack of sleep can result in impaired concentration and lengthened reaction times whilst awake, as well as a general feeling of tiredness. Difficulties in falling asleep can be caused by an inability to ignore conscious thoughts, which may be caused by stress or anxiety.

The respiratory rate of a subject or user during sleep can offer useful information about sleep quality. A number of existing systems are known for determining a user's respiratory rate. For example:
WO2016/027086 (SHEFFIELD HALLAM UNIVERSITY) discloses apparatus which calculates a user's respiratory rate from detecting a change in velocity of air flow in an airflow tunnel. However, the apparatus is intended for use in clinical or sports environments and is unsuitable for use during sleep.
US8562526 (RESMED/Heneghan et al) discloses apparatus which uses a RF signal reflected back from a subject to determine respiratory rate of a sleeping user.
US4202353 (HIRSCH) discloses a respiration sensing probe either in contact with the user (in mouth) or held in the user's nasal air stream.
US2017/0367651 (FACESENSE) discloses wearable apparatus which uses an array of four "thermal cameras" to collect thermal measurements from which respiration data can be calculated.

### SUMMARY OF THE INVENTION

According to an aspect of the invention, there is provided apparatus for determining respiratory information about a user, the apparatus comprising:
a. a plurality of temperature sensors, each temperature sensor having a field of view, the temperature sensors being arranged to detect a temperature gradient within a volume of interest and each providing an output signal;
b. a support frame comprising a head on which said temperature sensors are mounted, the support frame and temperature sensors making no contact, in use, with the user; and
c. a processor for processing one or more of said output signals to determine said respiratory information about the user.

The apparatus has the advantage that no contact with the user is required, so there is no disturbance or disruption to the user's normal sleep pattern. The plurality of fields of view of the temperature sensors enable a temperature gradient to be detected no matter the exact position of the user's head, which of course is likely to move during the period of sleep. No intervention or action is required by a clinician in order to use the apparatus and no clinician is required to be present during the user's sleep.

In an example, said temperature sensor is any one of a thermopile, a thermocouple, a pyrometer, an infrared camera, a thermistor, a resistance temperature detector, or a combination thereof

The temperature gradient preferably includes at least two peaks in temperature and the processor determines said respiratory information from a detectable temporal or physical separation of said two peaks.

In an example, the apparatus further comprises a tracker capable of tracking positional information about the user, in real-time. The apparatus may further comprise a closed-loop feedback control system capable of moving said support frame when the user's head moves outside said fields of view to reinstate the user's head within at least one of said fields of view. If, during sleep, the user moves so much that their head is no longer within any field of view of the temperature sensors, the support frame can be moved, in two dimensions to a new position, in which the user's head is back within at least one of the fields of view. This facilitates minimal interruption in the real-time collection of data.

Preferably, the temperature gradient is generated by exhalation by a recumbent user. The plurality of temperature sensors may be arranged such that their fields of view overlap. Overlapping fields of view mean better quality of data obtained. The processor may select any one of the temperature sensors' output signals from which to determine said respiratory information, based on (for example) which signal is strongest and/or most reliable. The processor may compare one or more of said non-selected output signals with said selected output signal.

The plurality of temperature sensors may have a focal point determined by the shape of the head of the support frame.

In an example, the temperature sensors operate at a sampling rate of 125Hz.

The apparatus may further comprise a camera, for example a charge-coupled device (CCD) camera, an infrared camera and/or a thermal imaging camera, capable of monitoring a surface temperature of the user and/or a temperature profile of the volume of interest.

The apparatus may further comprise a microphone and/or a movement sensor and/or facial recognition hardware and/or software. Recording sound associated with sleep may provide useful secondary data. Facial recognition may be useful to facilitate identification of the user, particularly when the apparatus is intended to be used regularly over a long period of time in order to monitor patterns in sleep behaviour and/or when data from many users is collated and processed.

In an example, the plurality of temperature sensors comprises four temperature sensors arranged in a generally square or rectangular configuration on said support frame.

The apparatus may comprise one or more sensors for monitoring ambient characteristics inside and/or outside said volume of interest. This secondary data may be useful to determine what effect room temperature, for example, has on the user's sleep pattern.

In an example, the temperature sensors are mountable at least 15cm from the user's face. Operating at this distance, there is no disturbance or disruption to the user's normal sleep pattern.

The support frame may comprise a support arm and a head on which the temperature sensors are mounted, the head being positionable and height-adjustable above the user's face when the user is recumbent.

The apparatus may comprise one or more sensors for monitoring physiological parameters of a user.

In another aspect, there is provided a method of sensing thermal information from a recumbent user, the method comprising the steps of:
a. providing apparatus or a monitoring system as described in any of the preceding paragraphs;
b. obtaining output signals from the plurality of temperature sensors; and
c. processing said output signals using said processor.

The method may further comprise the step of determining a respiratory rate of the user from the processed output signals.

The method may further comprise tracking positional information about the user, in real-time, and moving said support frame when the user's head moves outside said fields of view to reinstate the user's head within at least one of said fields of view.

Within the scope of this application it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible. The applicant reserves the right to change any originally filed claim or file any new claim accordingly, including the right to amend any originally filed claim to depend from and/or incorporate any feature of any other claim although not originally claimed in that manner.

### BRIEF DESCRIPTION OF THE DRAWINGS

One or more embodiments of the invention will now be described by way of example only, with reference to the accompanying drawings, in which:
Figure 1 is a perspective view of apparatus for determining respiratory information about a sleeping user;
Figure 2 shows the head of the support frame, drawn to a larger scale;
Figure 3 is a schematic representation of the overlapping fields of view of the thermopiles; and
Figure 4 is a schematic representation of the user exhaling into the volume of interest and showing the sensors' focal point F.

### DETAILED DESCRIPTION

Referring to the Figures, an example apparatus 1 for determining respiratory information about a user 2 is illustrated. The user 2 may be asleep, but the apparatus is equally capable of obtaining information from a non-sleeping user.

The apparatus 1 comprises a support frame 3 having a head 4 mounted on an arm 5 which, in turn, is mounted on a base unit 6.

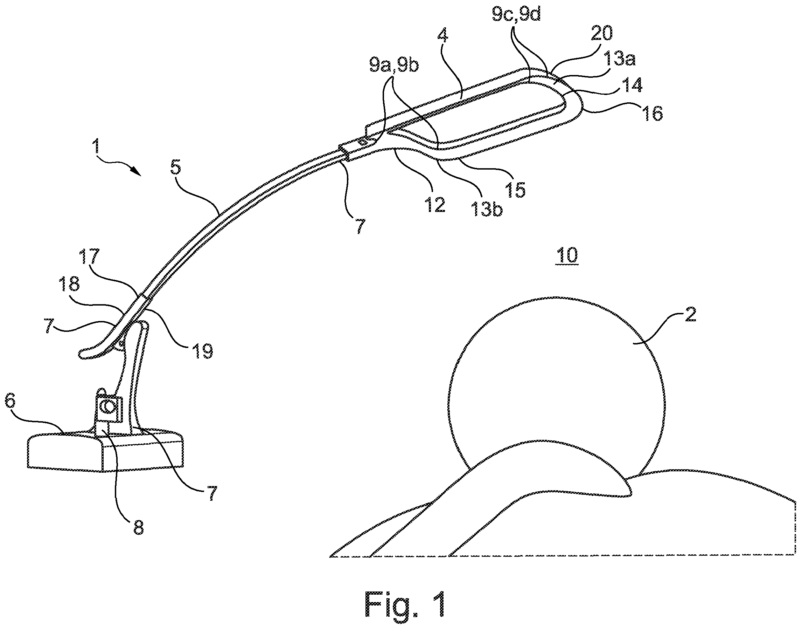

The angle, position and/or height of the arm 5 with respect to the base unit 6 and the position and/or angle of the head 4 with respect to the arm 5 is adjustable in order to best position the head 4 generally above the head of the recumbent user 2. Position adjustors 7 are indicated in Figure 1.

The base unit 6 is of a suitable size and shape to be placed on a bedside cabinet and includes a dock for a trigger device unit 8 of the type described in GB2511884B and/or GB2556973. The base unit 6 includes a processor, capability for wireless connectivity and a charging/audio jack.

The head 4 of the support frame is generally rectangular, although the head may be square and other shapes are possible. Temperature sensors, in this example thermopile sensors 9a, 9b, 9c, 9d are positioned in the corners of the support frame head 4. A suitable thermopile sensor is the ZTP-135BS Amphenol Thermometrics thermopile IR sensor.

There is a volume of interest 10 between the user's mouth/nose and the head 4 of the support frame. The volume of interest can be further defined by the volume immediately adjoining the mouth/nose and may include the user's head, neck, shoulders and/or pillow on which the user's head is positioned. The volume of interest is the volume through which exhaled air from the user travels and from which exhaled air the apparatus determines respiratory information about the user.

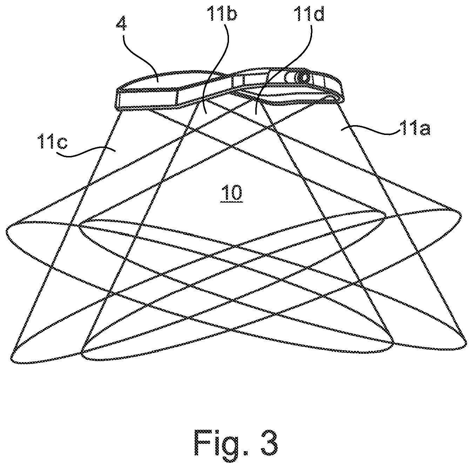

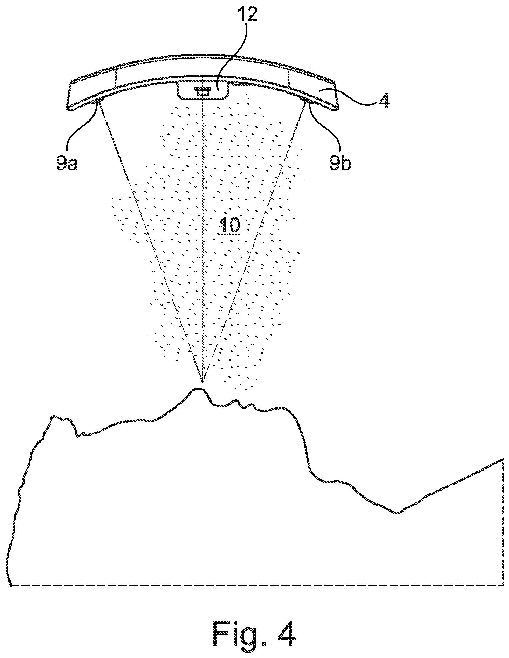

The thermopiles 9a, 9b, 9c, 9d each have a field of view 11a, 11b, 11c, 11d, represented in Figure 3, wherein it can be seen that the fields of view are generally cone shaped and overlapping one another. The overlapping fields of view encompass the volume of interest 10. With reference to Figure 4, the thermopiles 9a, 9b, 9c, 9d (and preferably others of the sensors in the head 4 of the support frame) have a focal point F near the centre of the user's face. The focal point F may be determined by the shape of the support frame head 4. The distance between a thermopile and the focal point F may be 250mm, for example.

The thermopiles 9a, 9b, 9c, 9d detect temperature gradients and output a voltage proportional to the detected change in temperature. Time domain traces from the thermopiles are used to determine respiration rate.

Exhaled air, moving away from the user through the volume of interest 10 after a breath will decrease in temperature, starting from a detectable peak. The next exhaled breath will generate a new peak in temperature with a trailing profile of decreasing temperature. The thermopiles enable peak to peak measurements to be made. Since each peak represents the start of an exhaled breath, information about the user's respiratory rate can be determined in real time. In addition to respiratory rate, respiratory volume may be estimated from the speed with which the peak of an exhaled breath reaches the thermopiles; a fast change in temperature indicates a bigger volume per breath and a slow change in temperature indicates a smaller volume per breath.

Each one of the thermopiles is sampled at up to 125 times per second i.e. a sampling frequency of 125 Hz. The maximum possible human respiratory rate (in extreme physical cases) is in the region of 75 breaths per minute. During sleep, respiratory rates are more likely to be in the range of 12 to 20 breaths per minute.

However, using 75 breaths per minute as the upper measurable respiration rate, this represents a maximum breathing frequency of 1.25Hz (i.e. 1.25 breaths per second).

To digitise these frequencies through analogue to digital conversion, oversampling is needed in order to get adequate resolution to post process the time domain traces and also to analyse in the frequency domain if necessary. Therefore, the claimed apparatus is capable of oversampling by up to 100 times, i.e. a sampling frequency of 125 Hz. A lower sampling rate may be used, depending on individual requirements.

Sampling at these higher frequencies may use local computation via dedicated microcontrollers. Correlation techniques can be used to extract breathing rates from the thermopiles' individual data streams

In addition to the data obtained from the thermopiles, useful secondary data can be obtained by the system that can be used in conjunction with the thermopile data to improve accuracy and confidence in the respiratory information determined. Such secondary data can be physiological parameters (e.g. movement of the user from a wearable accelerometer), ambient characteristics (e.g. pollution determined from a VOC sensor) as well as any other secondary data from sensors mounted on the support frame. Another potential source of secondary data is the trigger device unit 8, either docked on the base unit 8, or held in the user's hand. The trigger device unit 8 is described in more detail in GB2511884B and/or GB2556973.

Based on the shapes of the output data curves (amplitude, period etc), useful information can be determined about the user's breathing rate, time of sleep onset, sleep stage, possible sleep disorder or illness (sleep apnoea, flu or COPD for example). Over a longer period of time, data patterns can be monitored to determine the effects of illness or ageing.

Clearly, during sleep, the user may move such that they are no longer positioned face up and directly under the head 4 of the support frame. The overlapping fields of view 11a, 11b, 11c, 11d maximise the likelihood of at least one of the thermopiles still obtaining a good signal, even if the user moves to be positioned on their side, for example. In another embodiment (not illustrated), a closed-loop feedback control system is used to control motors which can move part or all of the support frame 3 when the user's head moves outside the fields of view to reinstate the user's head within at least one of the fields of view by repositioning the head 4 of the support frame.

An infrared camera 12 is mounted in the head 4 of the support frame which can obtain high frequency images to enhance or supplement the data provided by the thermopiles 9a, 9b, 9c, 9d. An infrared or thermal imaging camera can provide information about a temperature profile within the volume of interest 10. In addition, an infrared or thermal imaging camera can provide information about the surface temperature of the user's skin. For example, the extent to which capillaries near the skin's surface are visible may be related to respiratory rate.

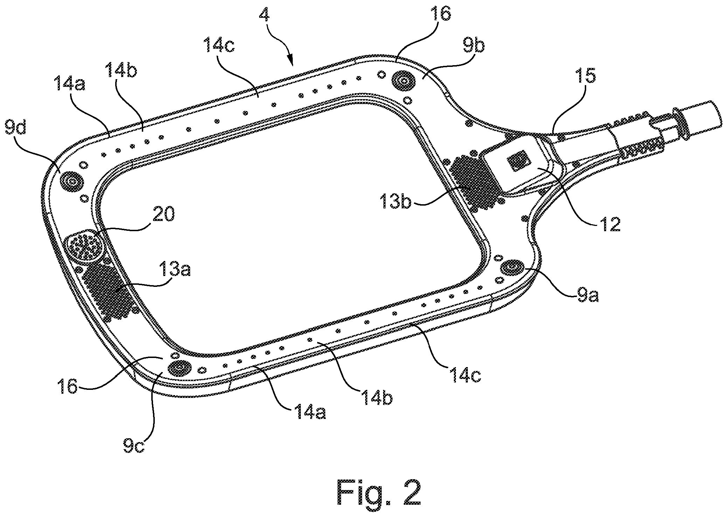

Audio or visual feedback may be provided to the user 2. This feedback may be the plurality of stimuli described in GB2511884, or other audio or visual stimuli. Speakers 13a, 13b and LEDs 14 are provided in the head 4 of the support frame to provide the stimuli. As shown in Figure 2, the LEDs may be a combination of blue light LEDS 14a, UV LEDs 14b and RGB LEDs 14c. An ambient light sensor 15 is also provided in the head 4 of the support frame.

Additional useful secondary data may be obtained from a microphone 16 in the head 4 of the support frame as to the user's breathing pattern, snoring etc.

Other sensors may be incorporated into the apparatus to measure ambient characteristics. As illustrated in Figure 1, the support arm 5 includes a barometric sensor 17, an air quality sensor 18 and a humidity and room temperature sensor 19. The head 4 of the support frame includes an alcohol sensor and/or VOC (volatile organic compound) sensor 20.

Throughout the description and claims of this specification, the term **"temperature sensor"** means any sensor whose output is dependent on detecting a thermal gradient. The thermal gradient may be caused by a change in ambient temperature and/or a change in the temperature of an airflow (for example an exhalation airflow) in which the thermopile may be situated. Examples of temperature sensors include any one or more of a thermopile, a thermocouple, a pyrometer, an infrared camera, a thermistor, a resistance temperature detector, or a combination thereof.

Throughout the description and claims of this specification, the term **"field of view"** of a temperature sensor means the volume in which the temperature sensor is capable of detecting a thermal gradient.

Throughout the description and the claims of this specification, the phrase **"ambient** characteristics" and variations thereof may comprise any one or more characteristics of an environment proximal to the apparatus. The ambient characteristic may comprise any one or more of, or any combination of any one or more of: humidity, levels of light, levels of natural light, levels of unnatural or artificial light, temperature local to the apparatus, ambient room temperature, pollution levels, oxygen levels and air pressure. It will be appreciated by the reader that the list of exemplary ambient characteristics is not exhaustive.

Throughout the description and claims of this specification the phrase **"physiological parameter"** and variations thereof mean any physical, biological, anatomical, medical or physiological, characteristic, or combination thereof, of the user. The physiological parameter may comprise, for example, any one or more of, or any combination of one or more of: heart rate, body temperature, skin temperature, galvanic skin response, blood oxygen saturation levels (SpO2), mixed venous oxygen saturation (SvO2), exerted grip force, reaction time or times, respiratory rate, blood pressure, movement, muscle contraction and/or relaxation, a composition or at least one or more constituents of exhaled air and electrical brain activity. It will be appreciated by the reader that the list of exemplary physiological parameters is not exhaustive.

It will be appreciated that embodiments of the present invention can be realised in the form of hardware, software or a combination of hardware and software. Any such software may be stored in the form of volatile or non-volatile storage such as, for example, a storage device like a ROM, whether erasable or rewritable or not, or in the form of memory such as, for example, RAM, memory chips, device or integrated circuits or on an optically or magnetically readable medium such as, for example, a CD, DVD, magnetic disk or magnetic tape. It will be appreciated that the storage devices and storage media are embodiments of machine-readable storage that are suitable for storing a program or programs that, when executed, implement embodiments of the present invention. Accordingly, embodiments provide a program comprising code for implementing apparatus or methods as described in any preceding paragraph and a machine-readable storage storing such a program. Still further, embodiments may be conveyed electronically via any medium such as a communication signal carried over a wired or wireless connection and embodiments suitably encompass the same.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of any foregoing embodiments. The claims should not be construed to cover merely the foregoing embodiments, but also any embodiments which fall within the scope of the claims.

### Reference numerals

- 1: Apparatus
- 2: User
- 3: Support frame
- 4: Support frame head
- 5: Support frame arm
- 6: Base unit
- 7: Position adjustors
- 8: Trigger device unit
- 9a, 9b, 9c, 9d: Thermopiles
- 10: Volume of interest
- 11a, 11b, 11c, 11d: Fields of view of thermopiles
- 12: Infrared camera
- 13a, 13b: Speakers
- 14: Feedback LEDs/IR
- 14a: blue light LEDs
- 14b: UV LEDs
- 14c: RGB LEDs
- 15: Ambient light sensor
- 16: Microphone
- 17: Barometric sensor
- 18: Air quality sensor
- 19: Humidity and room temperature sensor
- 20: Alcohol and/or VOC sensors
- F: sensors' focal point

## Claims

1. Apparatus (1) for determining respiratory information about a user (2), the apparatus comprising:
a. a plurality of temperature sensors (9a, 9b, 9c, 9d), each temperature sensor having a field of view (11a, 11b, 11c 11d), the temperature sensors (9a, 9b, 9c, 9d) being arranged to detect a temperature gradient within a volume of interest (10) and each providing an output signal;
b. a support frame (3) comprising a head (4) on which said temperature sensors (9a, 9b, 9c, 9d) are mounted, the support frame (3) and temperature sensors (9a, 9b, 9c, 9d) making no contact, in use, with the user (2); and
c. a processor for processing one or more of said output signals to determine said respiratory information about the user (2).

2. The apparatus (1) of claim 1 wherein said temperature gradient includes at least two peaks in temperature and wherein said processor determines said respiratory information from a detectable temporal or physical separation of said two peaks.

3. The apparatus (1) of claim 1 or claim 2 wherein said temperature sensor (9a, 9b, 9c, 9d) is any one of a thermopile, a thermocouple, a pyrometer, an infrared camera, a thermistor, a resistance temperature detector, or a combination thereof.

4. The apparatus (1) of any of the preceding claims comprising one or more of:
a) a tracker capable of tracking positional information about the user (2), in real-time;
b) a closed-loop feedback control system capable of moving said support frame (3) when the user's head moves outside said fields of view (11a, 11b, 11c 11d) to reinstate the user's head within at least one of said fields of view (11a, 11b, 11c 11d);
c) a camera (12), for example a CCD camera, an infrared camera and/or a thermal imaging camera, capable of monitoring a surface temperature of the user and/or a temperature profile of the volume of interest;
d) a microphone and/or a movement sensor.

5. The apparatus (1) of any of the preceding claims wherein said temperature gradient is generated by exhalation by a recumbent user (2).

6. The apparatus (1) of any of the preceding claims wherein the plurality of temperature sensors (9a, 9b, 9c, 9d) comprise one or more of the following properties:
a) are arranged such that their fields of view (11a, 11b, 11c 11d) overlap;
b) have a focal point determined by the shape of the head of the support frame (3);
c) comprise four temperature sensors (9a, 9b, 9c, 9d) arranged in a generally square or rectangular configuration on said support frame (3).

7. The apparatus (1) of any of the preceding claims wherein said temperature sensors (9a, 9b, 9c, 9d) operate at a sampling rate of 125Hz.

8. The apparatus (1) of any of the preceding claims comprising one or more of:
a) facial recognition hardware and/or software,
b) one or more sensors for monitoring ambient characteristics inside and/or outside said volume of interest (10).

9. The apparatus (1) of any of the preceding claims wherein the temperature sensors (9a, 9b, 9c, 9d) are mountable at least 15cm from the user's face.

10. The apparatus (1) of any of the preceding claims wherein the support frame comprises a support arm (5) and said head (4) on which the temperature sensors (9a, 9b, 9c, 9d) are mounted, the head (4) being positionable and height-adjustable above the user's face when the user is recumbent.

11. The apparatus (1) of any of the preceding claims wherein the processor selects one of said output signals from which to determine said respiratory information, and optionally wherein the processor compares one or more of said non-selected output signals with said selected output signal.

12. The apparatus (1) of any of the preceding claims further comprising one or more sensors for monitoring physiological parameters of a user.

13. A method of sensing thermal information from a recumbent user, the method comprising the steps of:
a. providing apparatus (1) as claimed in any of the preceding claims;
b. obtaining output signals from the plurality of temperature sensors (9a, 9b, 9c, 9d); and
c. processing said output signals using said processor.

14. The method of claim 13 further comprising the step of determining a respiratory rate of the user (2) from the processed output signals.

15. The method of claim 13 or claim 14 further comprising tracking positional information about the user (2), in real-time, and moving said support frame (3) when the user's head moves outside said fields of view (11a, 11b, 11c 11d) to reinstate the user's head within at least one of said fields of view (11a, 11b, 11c 11d).

## Patentansprüche

1. Vorrichtung (1) zum Bestimmen von Ateminformationen über einen Benutzer (2), wobei die Vorrichtung Folgendes umfasst:
a. eine Vielzahl von Temperatursensoren (9a, 9b, 9c, 9d), wobei jeder Temperatursensor ein Sichtfeld (11a, 11b, 11c, 11d) aufweist, wobei die Temperatursensoren (9a, 9b, 9c, 9d) angeordnet sind, um einen Temperaturgradienten innerhalb eines Volumens von Interesse (10) zu erfassen und jeweils ein Ausgangssignal bereitstellen;
b. einen Tragrahmen (3), der einen Kopf (4) umfasst, an dem die Temperatursensoren (9a, 9b, 9c, 9d) montiert sind, wobei der Tragrahmen (3) und die Temperatursensoren (9a, 9b, 9c, 9d) in Verwendung keinen Kontakt mit dem Benutzer (2) haben; und
c. einen Prozessor zum Verarbeiten von einem oder mehreren der Ausgangssignale, um die Ateminformationen über den Benutzer (2) zu bestimmen.

2. Vorrichtung (1) nach Anspruch 1, wobei der Temperaturgradient zumindest zwei Temperaturspitzen beinhaltet und wobei der Prozessor die Ateminformationen aus einer erfassbaren zeitlichen oder physischen Trennung der zwei Spitzen bestimmt.

3. Vorrichtung (1) nach Anspruch 1 oder Anspruch 2, wobei der Temperatursensor (9a, 9b, 9c, 9d) ein beliebiges von einer Thermosäule, einem Thermoelement, einem Pyrometer, einer Infrarotkamera, einem Thermistor, einem Widerstandstemperaturdetektor oder einer Kombination davon ist.

4. Vorrichtung (1) nach einem der vorhergehenden Ansprüche, umfassend eines oder mehrere von:
a) einem Verfolgungsgerät, das in der Lage ist, Positionsinformationen über den Benutzer (2) in Echtzeit zu verfolgen;
b) einem Rückkopplungssteuersystem mit geschlossenem Regelkreis, das in der Lage ist, den Tragrahmen (3) zu bewegen, wenn sich der Kopf des Benutzers außerhalb der Sichtfelder (11a, 11b, 11c, 11d) bewegt, um den Kopf des Benutzers wieder in zumindest eines der Sichtfelder (11a, 11b, 11c 11d) zu bringen;
c) einer Kamera (12), zum Beispiel einer CCD-Kamera, einer Infrarotkamera und/oder einer Wärmebildkamera, die in der Lage ist, eine Oberflächentemperatur des Benutzers und/oder ein Temperaturprofil des Volumens von Interesse zu überwachen;
d) einem Mikrofon und/oder einem Bewegungssensor.

5. Vorrichtung (1) nach einem der vorhergehenden Ansprüche, wobei der Temperaturgradient durch Ausatmen durch einen liegenden Benutzer (2) erzeugt wird.

6. Vorrichtung (1) nach einem der vorhergehenden Ansprüche, wobei die Vielzahl von Temperatursensoren (9a, 9b, 9c, 9d) eine oder mehrere der folgenden Eigenschaften umfasst:
a) sind angeordnet, sodass sich ihre Sichtfelder (11a, 11b, 11c 11d) überlappen;
b) weisen einen Brennpunkt auf, der durch die Form des Kopfes des Tragrahmens (3) bestimmt ist,
c) umfassen vier Temperatursensoren (9a, 9b, 9c, 9d), die in einer im Allgemeinen quadratischen oder rechteckigen Konfiguration an dem Tragrahmen (3) angeordnet sind.

7. Vorrichtung (1) nach einem der vorhergehenden Ansprüche, wobei die Temperatursensoren (9a, 9b, 9c, 9d) mit einer Abtastrate von 125 Hz arbeiten.

8. Vorrichtung (1) nach einem der vorhergehenden Ansprüche, umfassend eines oder mehrere von:
a) Gesichtserkennungshardware und/oder -software,
b) einem oder mehreren Sensoren zum Überwachen von Umgebungseigenschaften innerhalb und/oder außerhalb des Volumens von Interesse (10).

9. Vorrichtung (1) nach einem der vorhergehenden Ansprüche, wobei die Temperatursensoren (9a, 9b, 9c, 9d) zumindest 15 cm von dem Gesicht des Benutzers montierbar sind.

10. Vorrichtung (1) nach einem der vorhergehenden Ansprüche, wobei der Tragrahmen einen Tragarm (5) und den Kopf (4) umfasst, an dem die Temperatursensoren (9a, 9b, 9c, 9d) montiert sind, wobei der Kopf (4) über dem Gesicht des Benutzers positionierbar und höhenverstellbar ist, wenn der Benutzer liegt.

11. Vorrichtung (1) nach einem der vorhergehenden Ansprüche, wobei der Prozessor eines der Ausgangssignale auswählt, aus dem die Ateminformationen zu bestimmen sind, und wobei optional der Prozessor eines oder mehrere von den nicht ausgewählten Ausgangssignalen mit dem ausgewählten Ausgangssignal vergleicht.

12. Vorrichtung (1) nach einem der vorhergehenden Ansprüche, ferner umfassend einen oder mehrere Sensoren zum Überwachen von physiologischen Parametern eines Benutzers.

13. Verfahren zum Messen von Wärmeinformationen von einem liegenden Benutzer, wobei das Verfahren die folgenden Schritte umfasst:
a. Bereitstellen einer Vorrichtung (1) nach einem der vorhergehenden Ansprüche;
b. Erhalten von Ausgangssignalen von der Vielzahl von Temperatursensoren (9a, 9b, 9c, 9d); und
c. Verarbeiten der Ausgangssignale unter Verwendung des Prozessors.

14. Verfahren nach Anspruch 13, ferner umfassend den Schritt des Bestimmens einer Atemfrequenz des Benutzers (2) aus den verarbeiteten Ausgangssignalen.

15. Verfahren nach Anspruch 13 oder Anspruch 14, ferner umfassend Verfolgen von Positionsinformationen über den Benutzer (2) in Echtzeit und Bewegen des Tragrahmens (3), wenn sich der Kopf des Benutzers außerhalb der Sichtfelder (11a, 11b, 11c, 11d) bewegt, um den Kopf des Benutzers wieder in zumindest eines der Sichtfelder (11a, 11b, 11c, 11d) zu bringen.

## Revendications

1. Appareil (1) pour déterminer des informations respiratoires concernant un utilisateur (2), l'appareil comprenant :
a. une pluralité de capteurs de température (9a, 9b, 9c, 9d), chaque capteur de température comportant un champ de vision (11a, 11b, 11c, 11d), les capteurs de température (9a, 9b, 9c, 9d) étant agencés pour détecter un gradient de température dans un volume d'intérêt (10) et fournissant chacun un signal de sortie ;
b. un cadre de support (3) comprenant une tête (4) sur laquelle lesdits capteurs de température (9a, 9b, 9c, 9d) sont montés, le cadre de support (3) et les capteurs de température (9a, 9b, 9c, 9d) n'entrant jamais en contact, en cours d'utilisation, avec l'utilisateur (2) ; et
c. un processeur pour traiter un ou plusieurs desdits signaux de sortie afin de déterminer lesdites informations respiratoires concernant l'utilisateur (2).

2. Appareil (1) selon la revendication 1, dans lequel ledit gradient de température comprend au moins deux pics de température et dans lequel ledit processeur détermine lesdites informations respiratoires à partir d'une séparation temporelle ou physique détectable desdits deux pics.

3. Appareil (1) selon la revendication 1 ou la revendication 2, dans lequel ledit capteur de température (9a, 9b, 9c, 9d) est l'un quelconque parmi une thermopile, un thermocouple, un pyromètre, une caméra infrarouge, une thermistance, un détecteur de température à résistance, ou une combinaison de ceux-ci.

4. Appareil (1) selon l'une quelconque des revendications précédentes comprenant un ou plusieurs parmi :
a) un traceur capable de suivre des informations de position concernant l'utilisateur (2), en temps réel ;
b) un système de commande par rétroaction en boucle fermée capable de déplacer ledit cadre support (3) lorsque la tête de l'utilisateur se déplace en dehors desdits champs de vision (11a, 11b, 11c, 11d) pour réintégrer la tête de l'utilisateur dans au moins l'un desdits champs de vision (11a, 11b, 11c, 11d) ;
c) une caméra (12), par exemple une caméra CCD, une caméra infrarouge et/ou une caméra d'imagerie thermique, capable de surveiller une température de surface de l'utilisateur et/ou un profil de température du volume d'intérêt ;
d) un microphone et/ou un capteur de mouvement.

5. Appareil (1) selon l'une quelconque des revendications précédentes, dans lequel ledit gradient de température est généré par l'expiration d'un utilisateur en position allongée (2).

6. Appareil (1) selon l'une quelconque des revendications précédentes, dans lequel la pluralité de capteurs de température (9a, 9b, 9c, 9d) comprennent une ou plusieurs des propriétés suivantes :
a) sont disposés de telle sorte que leurs champs de vision (11a, 11b, 11c, 11d) se chevauchent ;
b) ont un point focal déterminé par la forme de la tête du cadre de support (3) ;
c) comprennent quatre capteurs de température (9a, 9b, 9c, 9d) disposés selon une configuration globalement carrée ou rectangulaire sur ledit cadre de support (3).

7. Appareil (1) selon l'une quelconque des revendications précédentes, dans lequel lesdits capteurs de température (9a, 9b, 9c, 9d) fonctionnent à une fréquence d'échantillonnage de 125 Hz.

8. Appareil (1) selon l'une quelconque des revendications précédentes comprenant un ou plusieurs parmi :
a) un matériel et/ou logiciel de reconnaissance faciale,
b) un ou plusieurs capteurs pour surveiller les caractéristiques ambiantes à l'intérieur et/ou à l'extérieur dudit volume d'intérêt (10).

9. Appareil (1) selon l'une quelconque des revendications précédentes, dans lequel les capteurs de température (9a, 9b, 9c, 9d) peuvent être montés à au moins 15 cm du visage de l'utilisateur.

10. Appareil (1) selon l'une quelconque des revendications précédentes, dans lequel le cadre de support comprend un bras de support (5) et ladite tête (4) sur laquelle les capteurs de température (9a, 9b, 9c, 9d) sont montés, la tête (4) étant positionnable et réglable en hauteur au-dessus du visage de l'utilisateur lorsque l'utilisateur est en position allongée.

11. Appareil (1) selon l'une quelconque des revendications précédentes, dans lequel le processeur sélectionne l'un desdits signaux de sortie à partir duquel déterminer lesdites informations respiratoires, et facultativement dans lequel le processeur compare un ou plusieurs desdits signaux de sortie non sélectionnés audit signal de sortie sélectionné.

12. Appareil (1) selon l'une quelconque des revendications précédentes, comprenant en outre un ou plusieurs capteurs pour surveiller les paramètres physiologiques d'un utilisateur.

13. Procédé de détection d'informations thermiques provenant d'un utilisateur en position allongée, le procédé comprenant les étapes de :
a. fourniture d'un appareil (1) selon l'une quelconque des revendications précédentes ;
b. obtention de signaux de sortie provenant de la pluralité de capteurs de température (9a, 9b, 9c, 9d) ; et
c. traitement desdits signaux de sortie en utilisant ledit processeur.

14. Procédé selon la revendication 13, comprenant en outre l'étape de détermination d'une fréquence respiratoire de l'utilisateur (2) à partir des signaux de sortie traités.

15. Procédé selon la revendication 13 ou la revendication 14, comprenant en outre le suivi des informations de position concernant l'utilisateur (2), en temps réel, et le déplacement dudit cadre de support (3) lorsque la tête de l'utilisateur se déplace en dehors desdits champs de vision (11a, 11b, 11c, 11d) pour réintégrer la tête de l'utilisateur dans au moins l'un desdits champs de vision (11a, 11b, 11c, 11d).
